# EUROPEAN PATENT SPECIFICATION

(11) **EP 1 602 720 B1**
(45) Date of publication and mention of the grant of the patent: **03.11.2010**
(21) Application number: 04713206.3
(22) Date of filing: 20.02.2004
(51) Int. Cl.: C12N 15/09, C12P 21/02, C07K 14/435

(54) **PROCESS FOR PRODUCING HETEROLOGOUS PROTEIN IN E. COLI**
VERFAHREN ZUR HERSTELLUNG EINES HETEROLOGEN PROTEINS IN E. COLI
PROCÉDÉ DE PRODUCTION D'UNE PROTÉINE HÉTÉROLOGUE DANS E. COLI

(30) Priority: 05.03.2003 JP 2003058992
(43) Date of publication of application: 07.12.2005
(73) Proprietor: Juridical Foundation The Chemo-Sero-Therapeutic Research Institute, Kumamoto-shi, Kumamoto 860-8568 (JP)
(72) Inventor: KOYANAGI, Satoshi, Chemo-sero-therap. res.inst., Kikuchi-shi, Kumamoto-ken 869-1298 (JP); SUGAWARA, Keishin, Chemo-sero-therap. res.inst., Kikuchi-shi, Kumamoto-ken 869-1298 (JP); MIYATSU, Yoshinobu, Chemo-sero-therap. res. inst., Kikuchi-shi, Kumamoto-ken 869-1298 (JP); MURAKAMI, Toshio, Chemo-sero-therap. research inst, Kikuchi-shi, Kumamoto-ken 869-1298 (JP); MAEDA, Toshihiro, Chemo-sero-therapeut. res.inst., Kikuchi-shi, Kumamoto-ken 869-1298 (JP); MIZOKAMI, Hiroshi, Chemo-sero-therap. res.inst., Kikuchi-shi, Kumamoto-ken 869-1298 (JP)
(74) Representative: Vossius & Partner
(86) International application number: PCT/JP2004/002017
(87) International publication number: WO 2004/078965

(56) References cited:
- EP-A2- 0 685 560
- WO-A1-91/06653
- WO-A2-00/61724
- JP-A- 05 199 866
- JP-A- 06 007 186
- JP-A- 06 141 869
- JP-A- 09 040 583
- JP-A- 10 218 897
- JP-A- 2002 034 568
- ANDREWS B ET AL: "A tightly regulated high level expression vector that utilizes a thermosensitive lac repressor: production of the human T cell receptor V beta 5.3 in Escherichia coli." GENE. 5 DEC 1996, vol. 182, no. 1-2, 5 December 1996 (1996-12-05), pages 101-109, XP002388638 ISSN: 0378-1119
- SHI H ET AL: "Temperature-induced expression of phb genes in Escherichia coli and the effect of temperature patterns on the production of poly-3-hydroxybutyrate." JOURNAL OF BIOSCIENCE AND BIOENGINEERING. 2001, vol. 91, no. 1, 2001, pages 21-26, XP002388639 ISSN: 1389-1723
- GILADI H ET AL: "Enhanced activity of the bacteriophage lambda PL promoter at low temperature." PROCEEDINGS OF THE NATIONAL ACADEMY OF SCIENCES OF THE UNITED STATES OF AMERICA. 14 MAR 1995, vol. 92, no. 6, 14 March 1995 (1995-03-14), pages 2184-2188, XP002388640 ISSN: 0027-8424
- NISHIYAMA C ET AL: "Determination of three disulfide bonds in a major house dust mite allergen, Der f II." INTERNATIONAL ARCHIVES OF ALLERGY AND IMMUNOLOGY 1993, vol. 101, no. 2, 1993, pages 159-166, XP002472626 ISSN: 1018-2438
- YABUTA M. ET AL: 'Thermo-inducible expression of a recombinant fusion protein by Escherichia coli lac repressor mutants' J. BIOTECHNOL. vol. 39, no. 1, 21 February 1995, pages 67 - 73, XP004037077
- SITNEY K.C. ET AL: 'Use of a modified tryptophanase promoter to direct high-level expression of foreign proteins in E. coli' ANNALS NY ACAD. SCI. vol. 782, 15 May 1996, pages 297 - 310, XP002979354
- KOYANAGI S. ET AL: 'Large-Scale Production of Major House Dust Mite Allergen Der f 2 Mutant (C8/119S) in Escherichia coli' JOURNAL OF BIOSCIENCE AND BIOENGINEERING, ELSEVIER, AMSTERDAM, NL vol. 106, no. 4, 01 October 2008, pages 387 - 392, XP026149346 ISSN: 1389-1723

## Description

### TECHNICAL FIELD OF THE INVENTION

The present invention relates to a method for producing a heterologous protein which comprises transforming E. coli with an expression vector in which expression of a heterologous protein is under control of a trp promoter capable of inducing expression through temperature shift, culturing the resulting transformants (heterologous protein-producing E. coli) in a range of 20 to 32°C to allow cell proliferation, and then culturing the transformants at 35 to 40°C in the absence of an inducer to thereby allow for expression of said heterologous protein, wherein E. coli is HB101 strain, JM109 strain or TB1 strain.

### BACKGROUND OF THE INVENTION

E. coli is a host indispensable for producing a recombinant protein. A promoter to be used for expression of a heterologous protein in E. coli includes, for example, a promoter that acts in the presence of an inducer such as isopropyl-beta-thiogalactoside (IPTG), a promoter that induces expression through pH change, a promoter that induces expression through removal or reduction of a specific amino acid or a sugar to render a host under starved conditions, and a promoter that induces expression through temperature shift (e.g. SAVVAS C. MAKRIDES Microbiological Reviers, Vol. 60, No. 3, p. 512-538, Sept. 1996).

Approach using an inducer is not preferable for industrial production of a heterologous protein of interest. Most of an inducer is a metabolic inhibitor and hence it is apprehended that its remaining may affect the living body. It is also troublesome to aseptically add an inducer to the culture in balance with proliferation of cells. Besides, the use of an expensive inducer will lead to a high cost for production. Thus, without the need of using an expensive inducer, it is desired to develop a method for producing a heterologous protein in a safe and simple manner at a low cost.

Among such desired methods as described above is an expression system where expression is induced through temperature shift with no use of an inducer. For a promoter capable of inducing expression through temperature shift, lac, trc, tac, PL, T7, λPL, PL-9G-50 and cspA promoters have been reported.

However, culture of E. coli cells in industrial production is usually aeration culture using a large-scale fermenter. In order to increase an expression level of a desired product, approach where expression is induced under low temperature has been exploited so that proliferation of cells may be suppressed while the desired product may preferentially be accumulated. This approach includes, for instance, recombinant E. coli cells that are constructed so that a β-galactosidase protein may be expressed under control of PL-9G-50 or cspA promoter (e.g. Hilla Giladi et al., Proc. Natl. Acad. Sci. USA Vol. 92, pp. 2184-2188, March 1995). In this approach, an expression level of the product is increased as a consequence of temperature shift but proliferation of cells is scarcely observed. As such, culture at low temperature tends to prolong time for culture due to a decreased metabolic rate of cells per se and hence is not efficient. In addition, prolonged time for culture will require additional necessity for maintenance of aseptic conditions to thereby induce higher risk in production. As described above, there are numerous problems to be solved in industrial production. For instance, Yuhki et al. reported that they successfully expressed a recombinant major mite allergen using E. coli (e.g. Japanese patent No. 2657861). However, their method uses IPTG as an inducer for expression of a major mite allergen and thus meets with the problem as described above.

It is known that Dermatophagoides is a major factor causing allergic diseases such as allergic asthma, allergic rhinitis, allergic dermatitis and allergic conjunctivitis. For therapy of allergic diseases, anti-allergic or anti-histaminic agent is used in general. This therapy, however, being a symptomatic treatment, is unable to radically cure allergic diseases. Hyposensitization where causative antigens of allergic diseases are injected is believed to be a sole measure for radical cure of allergic diseases. Hyposensitization is performed by injecting to a patient suffering from allergic diseases a small amount of allergens causative for said allergic diseases so that allergic reactions within the living body might disappear. Its efficacy is more than 70%.

Therapeutic allergens currently used for this purpose are those naturally extracted. For instance, allergen preparations for use in therapy of mite allergic diseases have been prepared from extracts of house dust. Extracts of house dust however contain not only mite allergens but also exceedingly many sorts of contaminants and hence producing preparations with a stable titer from such extracts would extremely be difficult. As a consequence, with allergic preparations that are produced from extracts of house dust as a starting material, there is always a risk of anaphylactic shock. In clinical scenes, a dose of allergic preparations has been altered with every change in lots of production of said allergic preparations, which is great burden for both physicians and patients. Besides, such allergic preparations are also problematic in that their production level is limited since their starting material is derived from a natural source.

In recent years, with progress of study in mite allergens, several major mite allergens (Der f 1, Der f 2, Der p 1, Der p 2, etc.) have already been identified (e.g. Platts-Mills et al., J. Allergy Clin. Immunol. 80, 755-775, 1987) and modified major mite allergens aiming at, for instance, reduction of anaphylactic shock has been developed.

For instance, a method for production of a modified major mite allergen is reported wherein prokaryotic or eukaryotic cells are transformed with an expression vector containing a gene encoding a modified Der f 2 in which a cysteine residue in natural Der f 2 is replaced with a serine residue and the resulting transformants are cultured so that a modified major mite allergen is produced from the culture (e.g. Japanese Patent Publication No. 253851/1994).

However, even with the method reported therein, IPTG is used for expression of a major mite allergen and thus there is a concern of effect to the living body.

### DISCLOSURE OF THE INVENTION

### (Technical Problem to be Solved by the Invention)

An object of the present invention is to provide a method for producing various heterologous proteins in a large amount in a safe and simple manner at a low cost without using an expensive inducer, which method does not rely upon extraction from a natural source but exploits the genetic recombination technique.

Described are heterologous proteins, especially major mite allergens, a secretory macrophage toxin from Actinobacillus pleuropneumoniae and a surface protective antigen (SpaA) of Erysipelothrix rhusiopathiae, that are produced by the method of the present invention and are completely free from an inducer.

### (Means for Solving the Problems)

In order to attain the objects described above, the present inventors have carried out intensive investigation and as a consequence found that a heterologous protein may be expressed by merely culturing heterologous protein-producing E. coli at high temperature without using an inducer, said heterologous protein-producing E. coli being prepared by the genetic recombination technique with a specific promoter, and that a heterologous protein may be expressed at a high expression level, while controlling cell proliferation, by first culturing said heterologous protein-producing E. coli at low temperature to a level of industrial production and subsequently culturing said E. coli at high temperature in the absence of an inducer.

Thus, the present invention relates to a method for producing a heterologous protein which comprises transforming E. coli with an expression vector in which expression of a heterologous protein is under control of a trp promoter capable of inducing expression through temperature shift, culturing the resulting transformants in a range of 20 to 32°C to allow for cell proliferation, and then culturing the transformants at 35 to 40°C in the absence of an inducer to thereby allow for expression of said heterologous protein, wherein E. coli is HB101 strain, JM109 strain or TB1 strain.

Described are heterologous proteins, especially major mite allergens, a secretory macrophage toxin from Actinobacillus pleuropneumoniae and a surface protective antigen of Erysipelothrix rhusiopathiae, that are produced by the method of the present invention and are completely free from an inducer.

It has not been reported that a promoter capable of inducing expression through temperature shift such as trp promoter as used in the method of the present invention may provide expression of a heterologous protein merely by culture at high temperature such as at 37 to 38°C without using an inducer.

### (More Efficacious Effects than Prior Art)

In accordance with the method of the present invention, various heterologous proteins may be provided in a large amount at a low cost without using an expensive inducer. Besides, in accordance with the method of the present invention, since expression may be induced merely by shifting culture temperature from low to high temperature, unlike a method using an inducer, there is no need for troublesome handling such as aseptic addition of an inducer to the culture in balance with proliferation of cells and hence maintenance of aseptic conditions is easy. Furthermore, in accordance with the method of the present invention wherein transformants are cultured in a range of 20 to 32°C and then shifted to culture at 35 to 40°C, cell proliferation may be controlled so that a variety of tasks (e.g. collection of cells and various assays during culture) may be performed in industrial production.

The heterologous proteins obtained in accordance with the method of the present invention are highly safe as being completely free from an inducer which bears concern of effect to the living body. A recombinant major mite allergens as exemplified for such heterologous proteins may be utilized for treatment or diagnosis of allergic diseases. A macrophage toxin (ApxIII) may be used as a vaccine to porcine pleuropneumonia whereas a surface protective antigen (ΔSpaA) of Erysipelothrix rhusiopathiae may be used as a vaccine to Erysipelothrix rhusiopathiae infection.

### BRIEF DESCRIPTION OF DRAWINGS

Fig. 1 shows construction of an expression vector pWU11-C8/119S.
Fig. 2 shows construction of an expression vector pFLU11-C8/119S.
Fig. 3 shows results of SDS-polyacrylamide gel electrophoresis of a sonicate of recombinant E. coli cells proliferated at 32°C in the presence of an inducer, in which Lane 1: pFLU11-CB/119S/JM109; Lane 2: pWU11-C8/119S/JM109; Lane 3: pFLU11-CB/119S/HB101; Lane 4: pWU11-C8/119S/HB101; Lane 5: pFLU11-C8/119S/LE392; Lane 6: pWU11-C8/119S/LE392; Lane 7: pFLU11-C8/119S/TB1; Lane 8: pWU11-C8/119S/TB1.
Fig. 4 shows results of SDS-polyacrylamide gel electrophoresis of a sonicate of recombinant E. coli cells proliferated at 37°C in the presence of an inducer, in which Lane 1: pFLU11-C8/119S/JM109; Lane 2: pFLU11-C8/119S/HB101; Lane 3: pFLU11-C8/119S/LE392; Lane 4: pFLU11-C8/119S/TB1; Lane 5: pWU11-C8/119S/JM109; Lane 6: pWU11-C8/119S/HB101; Lane 7: pWU11-C8/119S/LE392; Lane 8: pWU11-C8/119S/TB1.
Fig. 5 shows results of SDS-polyacrylamide gel electrophoresis of a sonicate of recombinant E. coli cells proliferated at 32°C in the absence of an inducer, in which Lane 1: pFLU11-C8/119S/JM109; Lane 2: pFLU11-C8/119S/HB101; Lane 3: pFLU11-C8/119S/LE392; Lane 4: pFLU11-C8/119S/TB1; Lane 5: pWU11-C8/119S/JM109; Lane 6: pWU11-C8/119S/HB101; Lane 7: pWU11-C8/119S/LE392; Lane 8: pWU11-C8/119S/TB1.
Fig. 6 shows results of SDS-polyacrylamide gel electrophoresis of a sonicate of recombinant E. coli cells proliferated at 37°C in the absence of an inducer, in which Lane 1: pFLU11-C8/119S/JM109; Lane 2: pFLU11-C8/119S/HB101; Lane 3: pFLU11-C8/119S/LE392; Lane 4: pFLU11-C8/119S/TB1; Lane 5: pWU11-C8/119S/JM109; Lane 6: pWU11-CB/119S/HB101; Lane 7: pWU11-C8/119S/LE392; Lane 8: pWU11-C8/119S/TB1.
Fig. 7 shows a proliferation curve of cells when cultured in a fermenter.
Fig. 8 shows results of SDS-polyacrylamide gel electrophoresis of a sonicate of recombinant E. coli cells proliferated in a fermenter culture, a sampling of which was performed with passage of time, in which Lane 1: before shifting to 37°C; Lane 2: 1 hour after the temperature shifting; Lane 3: 3 hours after the temperature shifting; Lane 4: 5 hours after the temperature shifting; Lane 5: 7 hours after the temperature shifting; Lane 6: 9 hours after the temperature shifting; Lane 7: 12 hours after the temperature shifting.
Fig. 9 shows construction of a plasmid pUC-Trp/Myc-His.
Fig. 10 shows construction of an expression vector pTrp-ApxIIIΔ2.
Fig. 11 shows results of SDS-polyacrylamide gel electrophoresis of a sonicate of ApxIIIΔ2-producing E. coli cells proliferated at 37°C in the absence of an inducer.
Fig. 12 shows staining with peroxidase-labeled anti-His tag antibody of PVDF membrane to which a sonicate electrophoresed on SDS-polyacrylamide gel is transferred wherein said sonicate is of ApxIIIΔ2-producing E. coli cells proliferated at 37°C in the absence of an inducer.
Fig. 13 shows construction of an expression vector pUC-Trp ΔspaA.

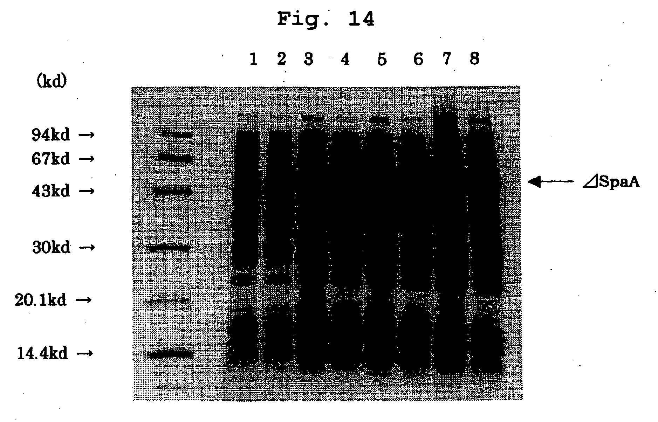

Fig. 14 shows results of SDS-polyacrylamide gel electrophoresis of a sonicate of ΔSpaA-producing E. coli cells that were proliferated at 30°C and of the proliferated ΔSpaA-producing E. coli cells that were further proliferated at 37°C in the absence of an inducer for induction of expression, in which Lane 1: HB101 cultured at 30°C for 15 hours; Lane 2: pUC - -TrpΔspaA/HB101 cultured at 30°C for 15 hours; Lane 3: HB101 cultured at 30°C for 15 hours, followed by shifting to culture at 37°C for 8 hours; Lane 4: pUC - TrpΔspaA/HB101 cultured at 30°C for 15 hours, followed by shifting to culture at 37°C for 8 hours; Lane 5: HB101 cultured at 30°C for 15 hours, followed by shifting to culture at 37°C for 12 hours; Lane 6: pUC - TrpΔspaA/HB101 cultured at 30°C for 15 hours, followed by shifting to culture at 37°C for 12 hours; Lane 7: HB101 cultured at 30°C for 15 hours, followed by shifting to culture at 37°C for 25 hours; Lane 8: pUC - TrpΔspaA/HB101 cultured at 30°C for 15 hours, followed by shifting to culture at 37°C for 25 hours.

### BEST MODE FOR CARRYING OUT THE INVENTION

The present inventors have transformed E. coli with an expression vector containing a replication origin of the vector pUC, trp promoter and a major mite allergen gene as a gene encoding a heterologous protein and have cultured the resulting recombinant E. coli at 32°C and 37°C in the absence of an inducer and as a consequence have found that a major mite allergen was scarcely expressed at 32°C while upon shifting to culture at 37°C a large amount of a major mite allergen could efficiently be produced. The present inventors have also found that the expression vector containing, as a gene encoding a heterologous protein, a gene encoding a secretory macrophage toxin (ApxIII) from Actinobacillus pleuropneumoniae or a gene encoding a surface protective antigen of Erysipelothrix rhusiopathiae may be used likewise so as to produce these proteins without the use of an inducer. As such, the present invention has been completed.

Accordingly, method of the present invention may be used for producing a major mite allergen which comprises transforming E. coli with an expression vector containing a replication origin of pUC, trp promoter and a major mite allergen gene, and culturing the resulting recombinant E. coli in the absence of an inducer at 32°C and then at 37°C, and the major mite allergen thus produced by said method that is completely free from an inducer. Likewise, the present invention encompasses the method as described in the claims where a gene encoding a secretory macrophage toxin from Actinobacillus pleuropneumoniae or a gene encoding a surface protective antigen of Erysipelothrix rhusiopathiae is used in place of a major mite allergen gene, and the thus produced secretory macrophage toxin from Actinobacillus pleuropneumoniae and a surface protective antigen of Erysipelothrix rhusiopathiae that are completely free from an inducer.

The method of the present invention is a method for producing a heterologous protein which comprises transforming E. coli with an expression vector in which a heterologous protein gene is under control of a trp promoter capable of inducing expression through temperature shift culturing the resulting recombinant E. coli (heterologous protein-producing E. coli) in a range of 20 to 32°C so that the recombinant cells are proliferated to an industrial level of production; and then culturing the resulting recombinant E. coli at 35 to 40°C in the absence of an inducer to allow for expression of said heterologous protein, wherein E. coli is HB101 strain, JM109 strain or TB1 strain. With the method of the present invention, a recombinant heterologous protein completely free from an inducer may be obtained in a large amount

In addition to a major mite allergen, a secretory macrophage toxin from Actinobacillus pleuropneumoniae and a surface protective antigen of Erysipelothrix rhusiopathiae, a heterologous protein that may be expressed in accordance with the method of the present invention includes, for instance, may be physiologically active substances such as a tissue plasminogen activator (tPA) and a human growth hormone, which have been used as a recombinant medicament and wherein absence of contaminants such as an inducer is desirable.

The present invention is explained in more detail hereinbelow for a major mite allergen.

Any of the major mite allergen genes hitherto identified including Der f 1, Der f 2, Der p 1 and Der p 2 may be used in the present invention. A nucleic acid fragment containing these major mite allergen genes may be prepared using as a starting material mRNAs or genomic DNAs extracted from mites in accordance with the common genetic recombination technique as described by Sambrook (Molecular Cloning, A Laboratory Manual Second Ed., Cold Spring Harbor Laboratory Press, N.Y., 1989).

In practice, commercially available kits may be used. For instance, for extraction of RNAs, reagents such as TRIzol reagent (Invitrogen), ISOGEN (NIPPON GENE CO., LTD.), and StrataPrep Total RNA Purification Kit (TOYOBO) may be used; for purification of mRNAs, kits such as mRNA Purification Kit (Amersham Bioscience), Poly(A) Quick mRNA Isolation Kit (TOYOBO), and mRNA Separator Kit (Clontech) may be used; for conversion into cDNAs, SuperScript plasmid system for cDNA synthesis and plasmid cloning (Invitrogen), cDNA Synthesis Kit (TAKARA SHUZO CO., LTD.), SMART PCR cDNA Synthesis & Library Construction Kits (Clontech), Directionary cDNA Library Construction systems (Novagen Inc.) may be used.

When a gene encoding a major mite allergen with a portion of its nucleotide sequence being replaced is used, a modified major mite allergen may be obtained. A nucleic acid fragment containing such a major mite allergen gene may be obtained by preparing a plasmid bearing cDNA of a modified major mite allergen and performing PCR using said plasmid as a template in accordance with the method described in Japanese Patent No. 2657861. In the method of the present invention, a nucleic acid fragment containing a modified major mite allergen gene is used.

Specifically, such a modified major mite allergen gene may be obtained by PCR using as a template a plasmid pFLT11-C8/119S bearing cDNA of a modified major mite allergen, a primer containing at its 5' end restriction enzyme site NspV (SEQ ID NO: 1) and a primer containing at its 3' end restriction enzyme site NruI (SEQ ID NO: 2). The thus obtained nucleic acid fragment encoding a modified major mite allergen (hereinafter also referred to as "C8/119S") is incorporated into an expression vector.

The expression vector used for the present invention may be constructed by linking the C8/119S fragment as described above downstream of a trp promoter capable of inducing expression through temperature shift (high temperature), which is then linked to a DNA fragment containing replication origin. Described are also trc promoter, PL promoter, T7 promoter, lac promoter, tac promoter and λPL promoter. Preferably, trp promoter is used as it may be usable for several strains of E. coli.

For replication origin, those derived from plasmids such as pBR322, pUC, pACYC, pSC101 and ColE1 for instance may be used. Preferably, replication origin derived from pUC is used since it is known to have a large copy number in a host (Molecular Cloning, A Laboratory Manual Second Ed., Cold Spring Harbor Laboratory Press, N.Y., 1989).

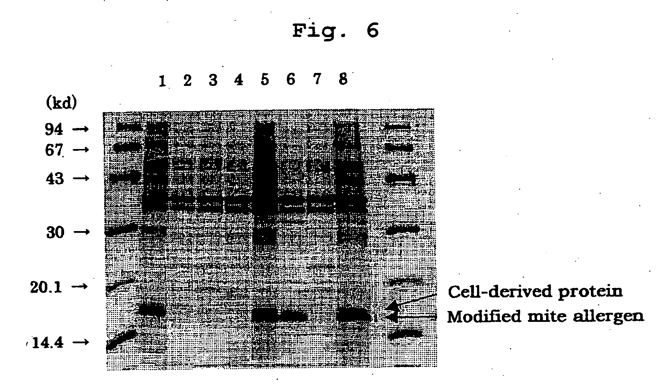

E. coli that may be transformed with the expression vector as described above may be HB101, JM109, and TB1. As will be shown in Example (Fig. 6) described below, an expression level of a major mite allergen may vary depending on a combination of a promoter and E. coli strain. In this regard, for a high expression level of a major mite allergen, a combination of a promoter and E. coli strain is significant. For instance, when trp promoter is used, any strain of HB101, JM109 or TB1 may be selected. When trc promoter may be used, JM109 strain may be selected. The highest and the most efficient expression of a major mite allergen may be obtained when E. coli HB101 or TB1 strain is transformed with an expression vector containing trp promoter, a major mite allergen (C8/119S) and replication origin derived from pUC.

Transformation of E. coli may be carried out using commercially available competent cells (Takara) in accordance with the instructions attached thereto. It may also be carried out as described in "Biotechnology Experiment Illustrated" (Shujunsha). Culture medium for culture of E. coli (e.g. LB, SOC, SOB may be used) and a reagent for selection of transformant (e.g. ampicillin) may be those commercially available. The pH of culture medium may be in a range that is suited for proliferation of E. coli (pH 6 to 8).

Recombinant E. coli expressing a major mite allergen may be screened as described below. E. coli cells cultured and proliferated in the presence or absence of an inducer are collected by centrifugation. To the collected cells is added a fixed amount of a saline to suspend the cells therein, which is then sonicated to disrupt the cells and subject to centrifugation (14,000 rpm, 30 minutes) to recover inclusion bodies. The recovered inclusion bodies are suspended in a suitable amount of a saline and then an amount of the suspension is applied to SDS-polyacrylamide gel electrophoresis. After Coomassie Brilliant Blue dye, expression of a major mite allergen is identified by a molecular size (about 15 kD) and a dye profile. An expression level is determined by measuring a wet weight of the recovered inclusion bodies. The thus obtained E. coli producing a major mite allergen is stocked in glycerol for a large-scale culture.

Identification of a major mite allergen (or detection of a major mite allergen) may also be done by the technique based on antigen/antibody reaction such as ELISA, Western blot or dot blot in addition to a molecular size as described above. These are all commonly used for detection of a heterologous protein expressed in E. coli and any of them may appropriately be used in accordance with the object.

One of the features of the present invention is that the E. coli producing a major mite allergen is first cultured and proliferated under conditions that do not allow for expression of a major mite allergen to an industrial scale of production and then the culture temperature is shifted to a higher temperature to thereby efficiently produce this protein without using an inducer. More specifically, the stocked E. coli cells in glycerol are inoculated in LB medium and cultured in a range of 20 to 32°C. This culture allows for proliferation of the cells but scarcely induces expression of a major mite allergen. When the number of the cells reached to an appropriate level, the culture temperature is shifted to 35 to 40°C without addition of an inducer and culture is continued. This temperature shift would accelerate expression of a major mite allergen. By culturing E. coli cells in a manner described above, both efficient proliferation of the cells in a culture medium even with deficient nutrients and a large-scale expression of a major mite allergen could be attained.

A culture temperature at which E. coli cells are proliferated may be in a range of 20 to 34°C, preferably in a range of 25 to 32°C. A temperature at which a major mite allergen is efficiently expressed may be in a range of 35 to 40°C, preferably 37 to 38°C, though it may vary depending on an expression vector used. The present invention is characterized by that the cells are first proliferated in a range of 20 to 32°C and then at 35 to 40°C so as to express a heterologous protein. However, it may also be possible to culture the cells at high temperature from the beginning so that both proliferation of the cells and expression of a heterologous protein might simultaneously be achieved.

A term of culture may vary depending on a culture temperature and a scale of production of a major mite allergen. Culture in a range of 20 to 32°C may preferably be continued until the recombinant E. coli cells are proliferated to the median of a logarithmic growth phase. Culture at 35 to 40°C may preferably be continued until an expression level of a major mite allergen reached its peak. More specifically, 10 ml of the recombinant E. coli (pWU11-C8/119S/HB101) glycerol stock is inoculated to about 1 L of culture medium. After culture at 32°C for 6 to 10 hours, the cells are then inoculated to 200 to 300 L of culture medium for culture at 25°C for 12 to 17 hours. Next, the cells are cultured at 37°C for 8 to 16 hours to produce about 7 to 10 g/L of inclusion bodies as a wet weight containing a major mite allergen.

For purifying a major mite allergen from E. coli producing a major mite allergen, a method for purification commonly used in the protein chemistry including, for instance, salting-out, ultrafiltration, isoelectric focusing, electrophoresis, ion exchange chromatography, gel filtration chromatography, affinity chromatography, hydrophobic chromatography, hydroxyapatite chromatography, may be used. From the practical point of view, due to possible contamination of a wide variety of cell debris, purification of a major mite allergen needs be carried out with a complicated combination of the techniques described above.

The above method for producing a major mite allergen is described as an exemplary of a method for producing a heterologous protein in E. coli. In place of a gene encoding a major mite allergen, a gene encoding another heterologous protein or a gene fragment thereof may also be used to allow for efficiently obtaining said protein or its partial oligopeptide or polypeptide free from an inducer. Such another protein gene includes a gene encoding a secretory macrophage toxin (ApxIII) from Actinobacillus pleuropneumoniae or a gene encoding a surface protective antigen of Erysipelothrix rhusiopathiae.

The present invention is explained in more detail by means of the following Examples but should not be construed to be limited thereto.

### Example 1: Preparation of E. coli producing major mite allergen

### (1) Construction of expression vector pWU11-C8/119S containing tryptophan (trp) promoter

First, a gene fragment was constructed in which a gene fragment of a major mite allergen (C8/119S) was bound downstream of trp promoter as described below.

A gene fragment of about 0.5 kbp containing trp promoter was obtained by complete digestion of E. coli plasmid ptrp ED 5-1 (Hallewell et al., Gene 9, 27-47, 1980) with HinfI. The cohesive ends of the resulting trp promoter gene fragment were filled, EcoRI linkers (TaKaRa) were added to the 5' and 3' ends and the obtained gene fragment was inserted at the EcoRI site of pBR322 to prepare a recombinant vector pW.

Next, a nucleic acid fragment containing a modified major mite allergen gene (C8/119S) was obtained by PCR using as a template a plasmid pFLT11-C8/119S (Japanese Patent No. 2657861) bearing said modified major mite allergen gene, a synthetic primer containing at its 5' end restriction enzyme site NspV (SEQ ID NO: 1) and a synthetic primer containing at its 3' end restriction enzyme site NruI (SEQ ID NO: 2).

A fragment obtained by complete digestion of this nucleic acid fragment with restriction enzymes NspV and NruI was linked with the recombinant vector pW completely digested with restriction enzymes ClaI and NruI with T4 ligase (TaKaRa) to prepare an expression vector pW11-C8/119S.

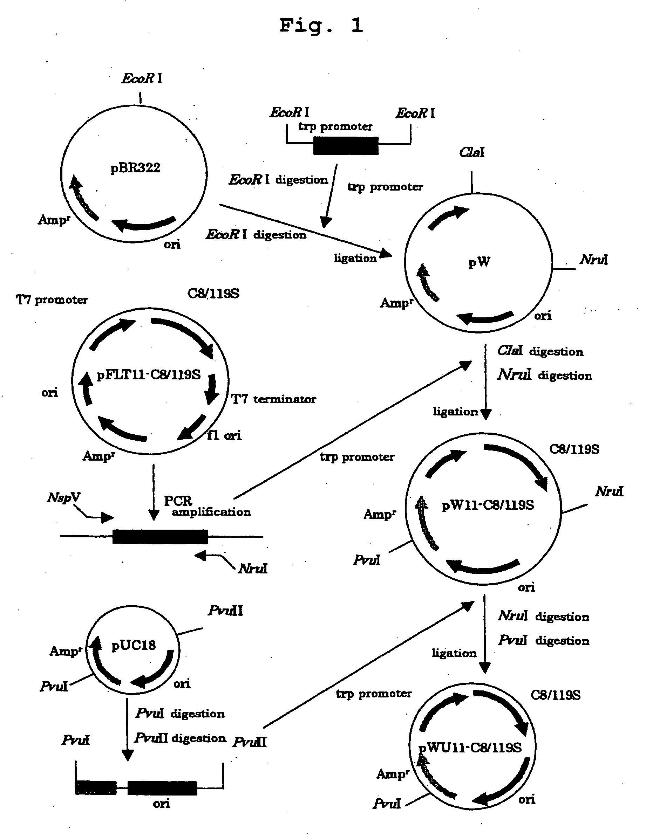

Next, for increasing an expression level, replication origin from pBR322 contained in pW11-C8/119S was replaced with that from pUC18. Plasmid pUC18 was completely digested with restriction enzymes PvuI and PvuII to obtain a gene fragment containing a desired replication origin. On the other hand, the plasmid pW11-C8/119S was completely digested with NruI and PvuI to obtain a nucleic acid fragment containing the modified major mite allergen gene (C8/1195). The thus prepared two nucleic acid fragments were linked together with T4 ligase to prepare an expression plasmid pWU11-C8/119S (Fig. 1).

### (2) Construction of expression vector pFLU11-C8/119S containing trp-lac fused promoter (trc)

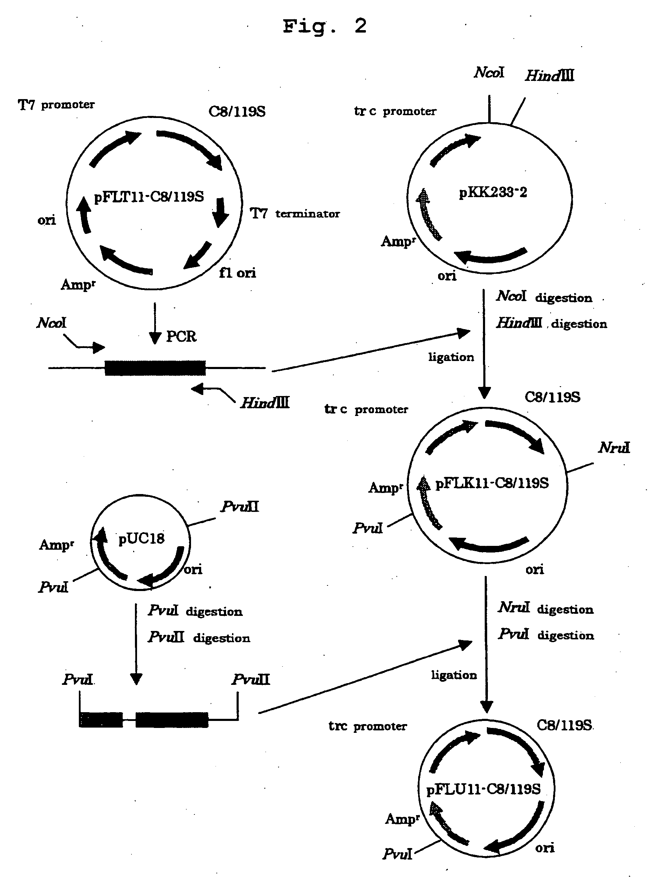

Trp-lac fused promoter, trc promoter, was prepared from an expression plasmid pKK233-2 (Amersham). The plasmid pKK233-2 was completely digested with restriction enzymes NcoI and HindIII to obtain a gene fragment of 4.6 kbp. A nucleic acid fragment containing a modified major mite allergen gene (C8/119S) was obtained by PCR as described above in step (1) except that a synthetic primer containing at its 5' end restriction enzyme site NcoI (SEQ ID NO: 3) and a synthetic primer containing at its 3' end restriction enzyme site HindIII (SEQ ID NO: 4) were used. A fragment obtained by complete digestion of this nucleic acid fragment with restriction enzymes NcoI and HindIII was linked with the gene fragment of 4.6 kbp containing trc promoter with T4 ligase to prepare an expression vector pFLK11-C8/119S. Replication origin from pBR322 contained in pFLK11-C8/119S was replaced with that of pUC18 as described above in step (1) to prepare an expression plasmid pFLU11-C8/119S (Fig. 2).

### (3) Preparation of recombinant E. coli

Each of E. coli strains HB101, JM109, TB1 and LE392 was transformed with either the expression plasmid pWU11-C8/119S or pFLU11-C8/119S obtained in step (1) and step (2) to obtain recombinant E. coli strains pWU11-C8/119S/HB101, pWU11-C8/119S/JM109, pWU11-C8/119S/TB1 and pWU11-C8/119S/LE392, and pFLU11-CB/119S/HB101, pFLU11-C8/119S/JM109, pFLU11-C8/119S/TB1 and pFLU11-C8/119S/LE392.

### Example 2: Screening of recombinant E. coli expressing major mite allergen

### (1) Expression of major mite allergen in the presence of inducer

Recombinant E. coli expressing a large amount of a major mite allergen in the presence of an inducer was screened as described below. To LB medium (10 ml) containing 50 µg/ml of ampicillin distributed to L-shaped test tubes were inoculated the recombinant E. coli strains pWU11-C8/119S/HB101, pWU11-C8/119S/JM109, pWU11-C8/119S/TB1, pWU11-C8/1195/LE392, pFLU11-C8/119S/HB101, pFLU11-C8/119S/JM109, pFLU11-C8/119S/TB1 and pFLU11-C8/119S/LE392. The inoculated recombinant E. coli strains were shake-cultured at 32°C for about 2 to 3 hours. When turbidity (OD 600nm) of the cells reached 0.4 or more, indoleacetic acid (Sigma) was added at a final concentration of 50 µg/ml for the expression system with trp promoter. For the expression system with trc promoter, isopropyl-β-D-galactopyranoside (Wako Pure Chemical Industries, Ltd.) was added at a final concentration of 1 mM. Subsequently, culture was further continued for 6 hours to induce expression.

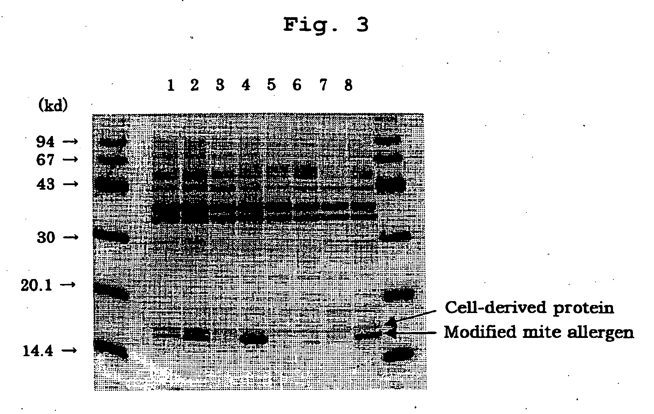

The obtained culture was centrifuged at 3,000 rpm for 10 minutes to collect the cells as a precipitate. The cells were resuspended in an appropriate amount of a saline to adjust the cell turbidity (OD 600nm) to 20. Each 1 ml of the cell suspension was put in sampling tubes (ASSIST), subject to sonication to disrupt the cells and centrifuged at 14,000 rpm for 30 minutes to recover inclusion bodies as a precipitate. The recovered inclusion bodies were resuspended in 1 ml of a saline. The suspension of the inclusion bodies was mixed with an equivalent amount of SDS sample buffer. After heating at 100°C for 2 minutes, the mixture was applied to SDS-polyacrylamide gel electrophoresis and dyed with Coomassie Brilliant Blue (nacalai tesque). Band profiles at around 15 kD were compared. As a consequence, it was found that a major mite allergen was expressed at the highest level in the recombinant strain pWU11-C8/119S/HB101, followed by the systems pWU11-C8/119S/JM109 and pWU11-C8/119S/TB1 in this order (Fig. 3).

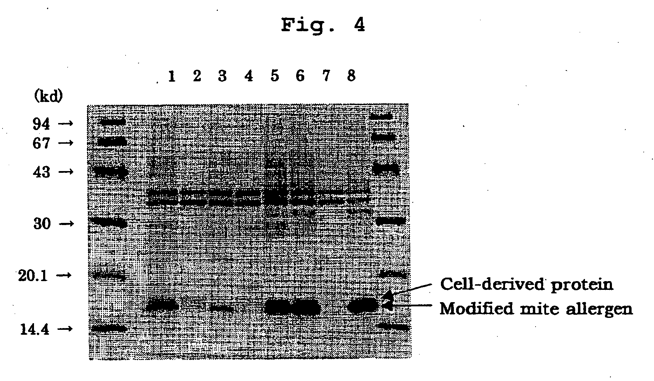

Next, an expression level of a major mite allergen was determined when cultured at 37°C. The same procedures as described above were used except that the cells were cultured at 37°C. As a result, it was found that a major mite allergen was expressed at a higher level in the recombinant strains pWU11-C8/119S/HB101 and pWU11-C8/119S/TB1, followed by the system pWU11-C8/119S/JM109 (Fig. 4).

### (2) Expression of major mite allergen in the absence of inducer

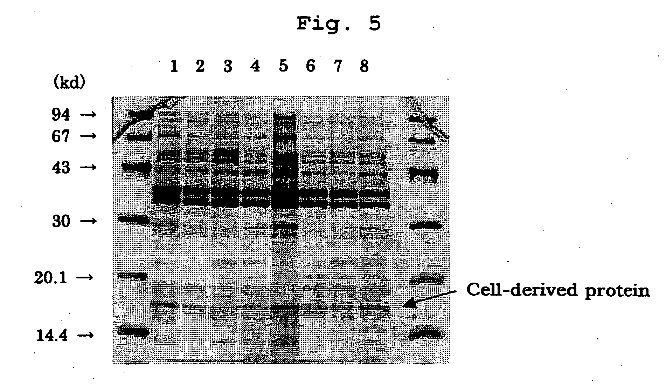

Recombinant E. coli expressing a large amount of a major mite allergen in the absence of an inducer was screened as described above in step (1). As a consequence, expression of a major mite allergen could scarcely been detected in any of the recombinant strains when shake-cultured at 32°C (Fig. 5) whereas the recombinant strains pWU11-C8/119S/HB101 and pWU11-C8/119S/TB1 produced a major mite allergen when shake-cultured at 37°C (Fig. 6). Likewise, expression of a major mite allergen could also be detected for pWU11-C8/119S/JM109 and pFLU11-C8/119S/JM109 though at a lower level than that of the above recombinant strains (Fig. 6). An expression level of a major mite allergen per cell was equivalent to that obtained using an inducer. However, proliferation of the cells (cell density) with no inducer was higher than that with an inducer by around 20 to 40% (Table 1). Therefore, it was found that an amount of a major mite allergen produced per culture volume in the absence of an inducer was higher than that obtained in the presence of an inducer.

**Table 1**

| Recombinant strains | Cell turbidity (OD 600nm) | | (Without)/(With) |
|---|---|---|---|
| | With | Without | |
| | inducer | | |
| pWU11-CB/119S/HB101 | 2.898 | 4.153 | 1.43 |
| pWU11-C8/119S/TB1 | 3.321 | 4.041 | 1.22 |
| pWU11-C8/119S/JM109 | 2.255 | 2.910 | 1.29 |
| pFLU11-C8/119S/JM109 | 2.547 | 3.132 | 1.23 |

### Example 3: Production of major mite allergen

The recombinant strain pWU11-C8/119S/HB101 was used as a strain producing a major mite allergen and its glycerol stock was prepared as described below. The recombinant cells were inoculated to 1 L of LB medium containing 50 µg/ml of ampicillin and shake-cultured at 32°C overnight. To this culture was added an equivalent amount of sterilized glycerol (Wako Pure Chemical Industries, Ltd.) and the mixture was thoroughly stirred. Each 10 ml of the mixture was delivered to 200 centrifuge tubes (ASSIST) and stored with lyophilization in a deep-freezer.

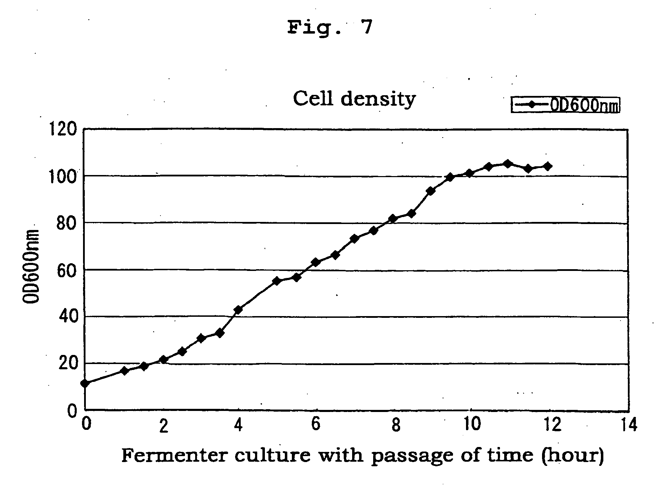

One glycerol stock was inoculated to LB medium (1.5 L) containing 50 µg/ml of ampicillin and shake-cultured at 32°C for 8 hours. This culture was inoculated to 250 L of LB medium and cultured at 25°C overnight with aeration. The culture temperature was raised to 37°C and culture was further continued for 12 hours with aeration (Fig. 7). While culture was continued, additives such as amino acids were supplemented in balance with proliferation of the cells.

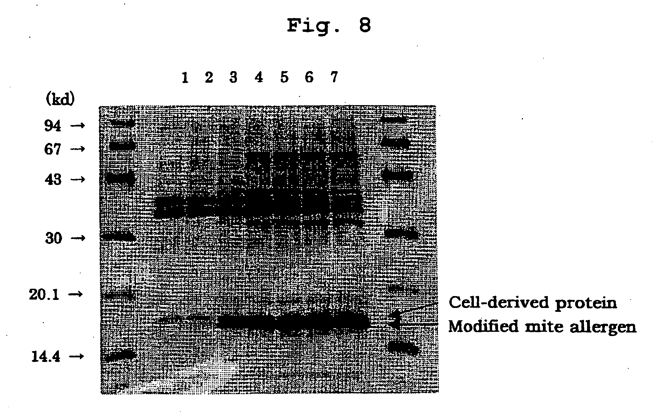

While culture was continued at 37°C, a portion of culture was sampled with passage of time. The sampled culture was centrifuged at 3,000 rpm for 10 minutes to collect the cells as a precipitate. The cells were resuspended in an appropriate amount of a saline to adjust the cell turbidity (OD 600nm) to 20. Each 1 ml of the cell suspension was put in sampling tubes (ASSIST), subject to sonication to disrupt the cells and centrifuged at 14,000 rpm for 30 minutes to recover inclusion bodies as a precipitate. The recovered inclusion bodies were resuspended in 1 ml of a saline. The suspension of the inclusion bodies was mixed with an equivalent amount of SDS sample buffer. After heating at 100°C for 2 minutes, the mixture was applied to SDS-polyacrylamide gel electrophoresis and dyed with Coomassie Brilliant Blue (nacalai tesque) to detect expression. As a result, it was found that a major mite allergen was accumulated as inclusion bodies three hours after the culture temperature was raised to 37°C. The cells continued to proliferate until termination of culture (Fig. 7) and an expression level of a major mite allergen per cell gradually increased (Fig. 8).

### Example 4: Preparation of recombinant E. coli producing macrophage toxin protein (ApxIIIΔ2) from Actinobacillus pleuropneumoniae

### (1) Construction of plasmid pUC-Trp/Myc-His

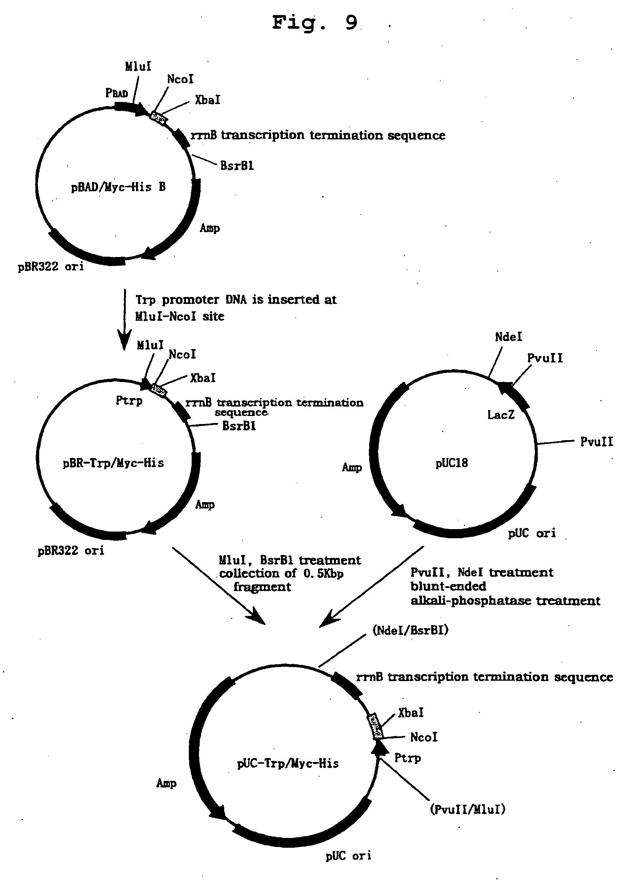

Plasmid pUC-Trp/Myc-His was constructed as described below bearing an expression cassette Trp/Myc-His for expression of a macrophage toxin protein (ApxIIIΔ2) under control of trp promoter (Fig. 9).
(a) The commercially available plasmid pBAD/Myc-His B (Invitrogen) was digested with restriction enzymes MluI and NcoI to purify a fragment of about 3.9 Kbp.
(b) An equivalent amount of synthetic Oligo DNAs (SEQ ID NOs: 5 and 6; entrusted Sigma-Genosis with synthesis thereof) corresponding to trp promoter regions of sense and antisense strands were mixed and the mixture was heated at 94°C for 1 minute and then at 75°C for 10 minutes, followed by gradual decrease in a temperature for annealing. Complementary strands for cohesive ends of MluI and NcoI were added upstream and downstream of the promoter, respectively.
(c) The annealed trp promoter DNA fragment (b) was ligated to the previously prepared 3.9 Kbp pBAD/Myc-HisB (a) with Ligation Kit Ver1 (TAKARA SHUZO CO., LTD.)(10°C, 24 hours). The ligation product was introduced into commercially available E. coli TOP10 (Invitrogen). The obtained plasmid was referred to as pBR-Trp/Myc-His.
(d) The plasmid pBR-Trp/Myc-His was treated with restriction enzymes MluI and BsrBI to purify a fragment of about 0.5 Kbp. This fragment contains an expression cassette Trp/Myc-His consisting of trp promoter, a multiple cloning site from pBAD/Myc-His, a DNA sequence of Histidine Hexamer and transcription termination sequences rrnB T1 and T2 from E. coli. This fragment of about 0.5 Kbp was blunt-ended with Blunting High Kit (TOYOBO).
(e) Plasmid pUC18 (TAKARA SHUZO CO., LTD.) was treated with restriction enzymes PvuII and NdeI to purify a fragment of about 2.2 Kbp and blunt-ended with Blunting High Kit (TOYOBO).
(f) The blunt-ended fragment of about 0.5 Kbp comprising trp promoter (d) was ligated to the pUC18 DNA that was treated with restriction enzymes and blunt-ended using Ligation Kit Ver1 (TAKARA SHUZO CO., LTD.)(10°C, 24 hours). The ligated product was introduced into E. col (JM109) and from the obtained transformant was purified plasmid pUC-Trp/Myc-His containing Trp/Myc-His (Fig. 9).

### (2) Construction of expression vector pTrp-ApxIIIΔ2

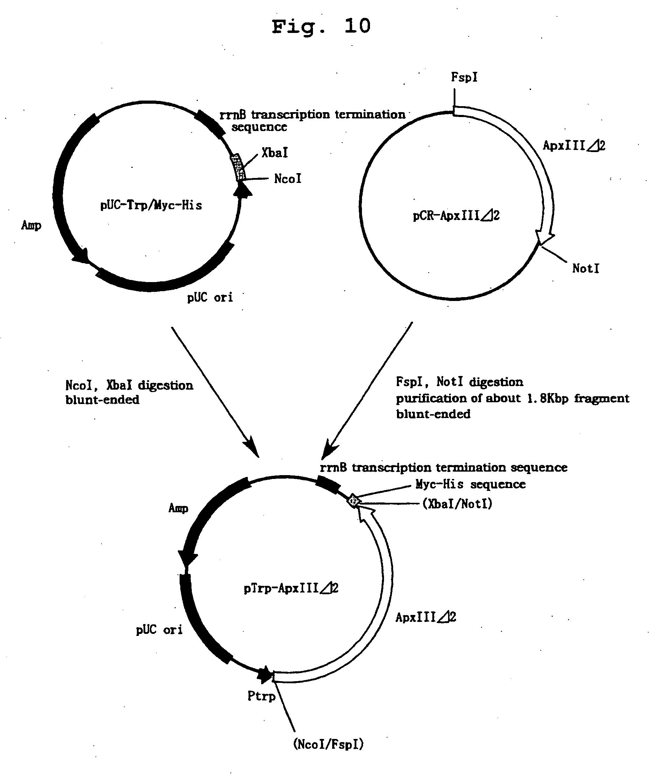

An expression vector pTrp-ApxIIIΔ2 was constructed as described below wherein a gene fragment encoding ApxIII excepting its N-terminal hydrophobic region (ApxIIIΔ2) was inserted into plasmid pUC-Trp/Myc-His (Fig. 10). Using DNA extraction kit ISOPLANT (Wako Pure Chemical Industries, Ltd.), DNAs were extracted from Actinobacillus pleuropneumoniae NG-22 strain (serum type 2). With the extracted DNAs as a template, a region encoding ApxIIIΔ2 was amplified using PCR Kit (TAKARA SHUZO CO., LTD.; LA Taq). For PCR, upstream primer (SEQ ID NO: 7) and downstream primer (SEQ ID NO: 8) were used in which FspI site or NotI site, respectively, was added to a nucleotide sequence selected on the basis of the nucleotide sequence of apxIIIA (GeneBank Accession No. L12145) as reported by Chang et al. (DNA Cell Biol 1993; 12: 351-62). The fragment obtained by PCR was cloned into the commercially available vector pCR2.1 TOPO (Invitrogen) to obtain pCR-ApxIIIΔ2. The obtained plasmid was treated with restriction enzymes FspI and NotI and a DNA fragment of about 1.8 Kbp was purified and blunt-ended with Blunting High Kit (TOYOBO). On the other hand, the plasmid pUC-Trp/Myc-His obtained in step (1) was treated with restriction enzymes NcoI and XbaI, blunt-ended with the same kit and treated with alkali phosphatase from shrimp (Promega). This DNA was ligated with the previously prepared DNA fragment of about 1.8 Kbp encoding ApxIIIΔ2 to construct an expression vector pTrp-ApxIIIΔ2 (Fig. 10).

### (3) Expression of ApxIIIΔ2 in the absence of inducer

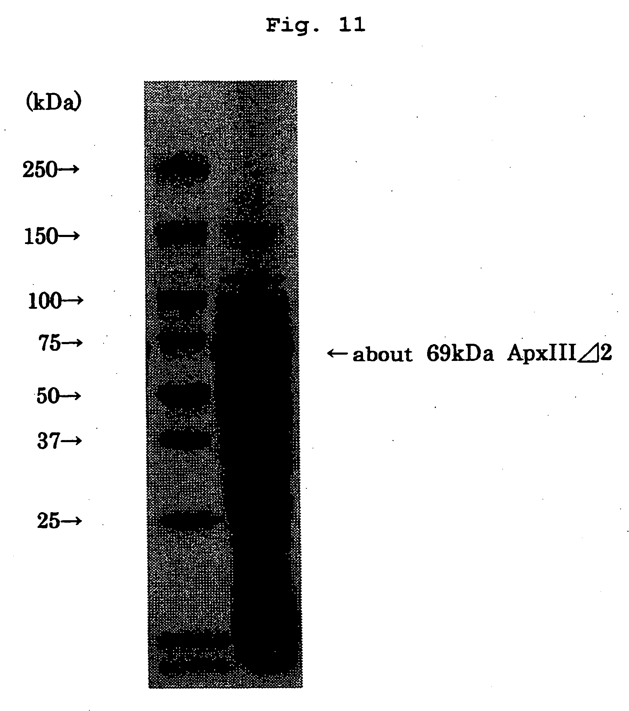

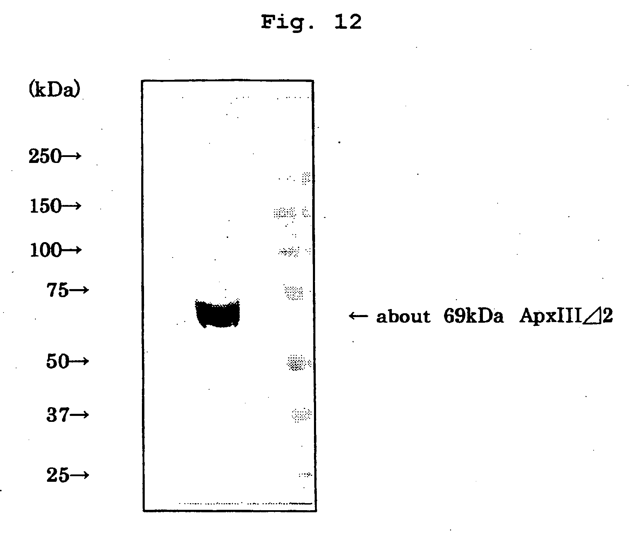

The expression vector pTrp-ApxIIIΔ2 as described above was introduced into E. coli HB101 (TAKARA SHUZO CO., LTD.) to obtain HB101 transformant having pTrp-ApxIIIΔ2. The transformant cells were inoculated to CIRCLEGROW (BI0101) and cultured at 37°C for 16 hours. The culture solution (1 ml) was centrifuged at 10,000 rpm for 1 minute to collect cells. The collected cells were mixed with 400 µl of SDS sample buffer. After heating at 100°C for 2 minutes, the mixture was subject to SDS-polyacrylamide gel electrophoresis and dyed with Coomassie Brilliant Blue (Fig. 11). Following electrophoresis, the expressed proteins were transferred to PVDF membrane, reacted with a peroxidase-labeled anti-His tag antibody (Invitrogen) at 4°C overnight and dyed with Konica Immunostein (SEIKAGAKU CORPORATION) (Fig. 12). Expression of ApxIIIΔ2 of about 69 kDa was detected.

### Example 5: Preparation of recombinant E. coli producing a surface protective antigen (ΔSpaA) of Erysipelothrix rhusiopathiae

### (1) Construction of expression vector pUC-Trp ΔspaA

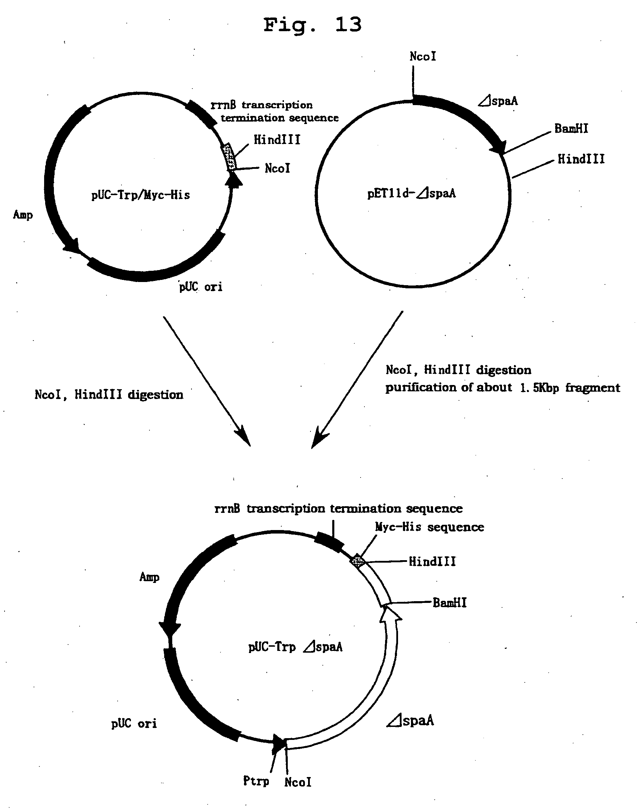

An expression vector pUC-Trp ΔspaA was constructed as described below wherein a gene fragment of 1206 bp (hereinafter referred to as "ΔspaA") is inserted into plasmid pUC-Trp/Myc-His and said fragment encodes a partial surface protective antigen of Erysipelothrix rhusiopathiae (hereinafter referred to as "ΔSpaA") encoded by the nucleotide sequence corresponding to No. 88 to No. 1293 nucleotides of a gene encoding a surface protective antigen (spaA) of Erysipelothrix rhusiopathiae (Fig. 13).

PCR was performed using as a template DNAs extracted from culture of Erysipelothrix rhusiopathiae SE-9 strain with ISOPLANT (NIPPON GENE CO., LTD.), two primers synthesized on the basis of information of the publicly known nucleotide sequences (Imada, Y., Goji, N., Ishikawa, H., Kishima, M. and Sekizaki, T. Truncated surface protective antigen (SpaA) of Erysipelothrix rhusiopathiae serotype 1a elicits protection against challenge with serotypes 1a and 2b in pigs: Infect. Immun. 67 (9), 4376-4382 (1999)/GeneBank Accession No. AB019124), i.e. upstream primer with NcoI site (SEQ ID NO: 9) and downstream primer with BamHI site (SEQ ID NO: 10). The amplified fragment containing ΔspaA was double-digested with restriction enzymes NcoI and BamHI and ligated with T4 DNA ligase to plasmid pET11d (Novagen) previously digested with the same restriction enzymes. E. coli BL21 (DE3) strain was transformed with the ligated DNA and the transformant cells were inoculated to LB agar containing 50 µg/ml of ampicillin to select ampicillin-resistant transformant. Plasmid was extracted from the selected transformant to obtain plasmid pET11d ΔspaA bearing ΔspaA (Fig. 13).

Next, the plasmid pUC-Trp/Myc-His obtained in Example 4(1) was double-digested with restriction enzymes NcoI and HindIII and then subject to 0.8% agarose gel electrophoresis to recover a nucleic acid fragment of about 2.7 kb containing trp promoter. On the other hand, pET11d ΔspaA was double-digested with restriction enzymes NcoI and HindIII and then subject to 0.8% agarose gel electrophoresis to recover a nucleic acid fragment of about 1.5 kb containing ΔspaA. This nucleic acid fragment was ligated to the above nucleic acid fragment of about 2.7 kb with T4 DNA ligase. E. coli HB101 strain was transformed with the ligated DNA and the transformant cells were inoculated to LB agar containing 50 µg/ml of ampicillin to select ampicillin-resistant transformant to obtain recombinant E. coli bearing the expression vector pUC-Trp ΔspaA (Fig. 13).

### (2) Expression of ΔSpaA in the absence of inducer

The recombinant E. coli cells bearing the expression vector pUC-Trp ΔspaA as described above were inoculated to CIRCLEGROW (BIO101) and cultured at 30°C for about 15 hours, then at 37°C for about 25 hours. E. coli (HB101) strain not transformed was used as control. An equivalent amount of the cultured cells sampled with passage of time and SDS sample buffer were mixed together. After heating at 100°C for 2 minutes, the mixture was subject to SDS-polyacrylamide gel electrophoresis and dyed with Coomassie Brilliant Blue. As a result, expression of ΔSpaA of about 50 kDa was detected after shifting the culture temperature from 30°C to 37°C (Fig. 14).

## Claims

1. A method for producing a heterologous protein which comprises transforming E. coli with an expression vector in which expression of a heterologous protein is under control of a trp promoter capable of inducing expression through temperature shift, culturing the resulting transformants in a range of 20 to 32°C to allow for cell proliferation, and then culturing the transformants at 35 to 40°C in the absence of an inducer to thereby allow for expression of said heterologous protein and wherein E. coli is HB101 strain, JM109 strain or TB1 strain.

2. The method according to claim 1, wherein the transformants are then cultured at 37 to 38°C in the absence of an inducer to thereby allow for expression of said heterologous protein.

3. The method according to claim 1 or 2, wherein the transformants are cultured in a range of 25 to 32°C to allow for cell proliferation.

4. The method according to any one of claims 1 to 3, wherein said heterologous protein is a major mite allergen, a secretory macrophage toxin from Actinobacillus pleuropneumoniae or a surface protective antigen of Erysipelothrix rhusiopathiae.

## Patentansprüche

1. Verfahren zur Herstellung eines heterologen Proteins umfassend das Transformieren von E. coli mit einem Expressionsvektor, in dem die Expression eines heterologen Proteins unter der Kontrolle eines trp-Promotors ist, der in der Lage ist, die Expression durch Temperaturveränderung zu induzieren, das Züchten der entstandenen Transformanten in einem Bereich von 20 bis 32°C, um Zellproliferation zu ermöglichen, und dann das Züchten der Transformanten bei 35 bis 40°C in der Abwesenheit eines Induktors, um dabei die Expression des heterologen Proteins zu ermöglichen und wobei E. coli der HB101-Stamm, JM109-Stamm oder TB1-Stamm ist.

2. Verfahren gemäß Anspruch 1, wobei die Transformanten dann bei 37 bis 38°C in der Abwesenheit eines Induktors gezüchtet werden, um dabei die Expression des heterologen Proteins zu ermöglichen.

3. Verfahren gemäß Anspruch 1 oder 2, wobei die Transformanten in einem Bereich von 25 bis 32°C gezüchtet werden, um Zellproliferation zu ermöglichen.

4. Verfahren gemäß einem der Ansprüche 1 bis 3, wobei das heterologe Protein ein Hauptallergen der Milbe, ein sekretorisches Makrophagentoxin von Actinobacillus pleuropneumoniae oder ein oberflächen-protektives Antigen von Erysipelothrix rhusiopathiae.

## Revendications

1. Méthode de production d'une protéine hétérologue qui comprend la transformation d'E. coli avec un vecteur d'expression dans lequel l'expression d'une protéine hétérologue est sous le contrôle d'un promoteur trp capable d'induire l'expression du fait d'un changement de température, la mise en culture des transformants résultants dans une gamme de 20 à 32°C pour permettre la prolifération cellulaire, et enfin la mise en culture des transformants à 35 jusque 40°C en l'absence d'un inducteur pour ainsi permettre l'expression de ladite protéine hétérologue et dans laquelle E. coli est une souche HB101, une souche JM109 ou une souche TB1.

2. Méthode selon la revendication 1, dans laquelle les transformants sont ensuite mis en culture de 37 à 38°C en l'absence d'un inducteur pour permettre ainsi l'expression de ladite protéine hétérologue.

3. Méthode selon la revendication 1 ou 2, dans laquelle les transformants sont mis en culture dans une gamme de 25 à 32°C pour permettre la prolifération cellulaire.

4. Méthode selon l'une quelconque des revendications 1 à 3, dans laquelle ladite protéine hétérologue, est un allergène majeur d'acarien, une toxine macrophagique sécrétée par Actinobacillus pleuropneumoniae ou un antigène protecteur de surface de Erysipelothrix rhusiopathiae.


### SEQUENCE LISTING

<110> JURIDICAL FOUNDATION THE CHEMO-SERO-THERAPEUTIC RESEARCH INSTITUTE
   <120> Method for the production of foreign proteins in E. coli
   <130> 664288
   <150> JP 2003-58992
   <151> 2003-03-05
   <160> 10
<210> 1
   <211> 30
   <212> DNA
   <213> Artificial Sequence
<220>
   <223> PCR primer
<400> 1
   cggttcgaat ggatcaagtc gatgttaaag 30
<210> 2
   <211> 30
   <212> DNA
   <213> Artificial Sequence
<220>
   <223> PCR primer
<400> 2
   gcttcgcgat cattaatcac ggattttagc 30
<210> 3
   <211> 27
   <212> DNA
   <213> Artificial Sequence
<220>
   <223> PCR primer
<400> 3
   gcgccatgga tcaagtcgat gttaaag 27
<210> 4
   <211> 30
   <212> DNA
   <213> Artificial Sequence
<220>
   <223> PCR primer
<400> 4
   gcgaagcttt cattaatcac ggattttagc 30
<210> 5
   <211> 86
   <212> DNA
   <213> Artificial Sequence
<220>
   <223> Trp promoter sequence (sense)
<400> 5
   cgcgtgctaa ttctgaaatg agctgttgac aattaatcat cgaactagtt aactagtacg 60
   caagttcacg taaaaagggt atcgac 86
<210> 6
   <211> 88
   <212> DNA
   <213> Artificial Sequence
<220>
   <223> Trp promoter sequence (antisense)
<400> 6
   catggtcgat acccttttta cgtgaacttg cgtactagtt aactagttcg atgattaatt 60
   gtcaacagct catttcagaa tgctagca 88
<210> 7
   <211> 35
   <212> DNA
   <213> Artificial Sequence
<220>
   <223> PCR primer
<400> 7
   tgcgcagctc gtcataaagc tttcttagaa gattc 35
<210> 8
   <211> 36
   <212> DNA
   <213> Artificial Sequence
<220>
   <223> PCR primer
<400> 8
   gcggccgcag ctgctctagc taggttacct agcatc 36
<210> 9
   <211> 37
   <212> DNA
   <213> Artificial Sequence
<220>
   <223> PCR primer
<400> 9
   catgccatgg ctttcgctga ttcgacagat atttctg 37
<210> 10
   <211> 33
   <212> DNA
   <213> Artificial Sequence
<220>
   <223> PCR primer
<400> 10
   cgcggatcct taatctttag gtttttcttc atc 33